# EUROPEAN PATENT APPLICATION

(11) **EP 4 635 575 A1**
(43) Date of publication of application: **22.10.2025**
(21) Application number: 25155377.2
(22) Date of filing: 31.01.2025
(51) Int. Cl.: A61P 35/02, C07K 16/18

(54) **USE OF ANTI-C4D MONOCLONAL ANTIBODY IN SECOND-LINE IMMUNOTHERAPY IN PATIENTS REFRACTORY TO FIRST-LINE IMMUNOTHERAPY**

(30) Priority: 16.04.2024 PL 44831124
(71) Applicant: Gdanski Uniwersytet Medyczny, 80-210 Gdansk (PL)
(72) Inventor: Budka, Daria, 64-920 Pila (PL); Okroj, Marcin, 80-299 Gdansk (PL)
(74) Representative: Godlewski, Piotr

(57) **Abstract**

The subject of the invention is a monoclonal anti-C4d antibody (mAb2) containing E430G and/or E345R substitutions for use in second-line immunotherapy in patients exhibiting resistance to the first-line immunotherapy.

List of sequences:

<110> Gdańsk Medical University

<120> Application of anti-C4d monoclonal antibody in second-line immunotherapy for patients showing resistance to first-line immunotherapy

<130> 62307

<160> 2

<170> BiSSAP 1.3.6

<210> 1

<211> 452

<212> PRT

<213> Artificial Sequence

<220>

<223> heavy chain of anty-C4d mAb2 antibody

<400> 1

Gln Ser Val Glu Glu Ser Gly Gly Arg Leu Val Thr Pro Gly Thr Pro

1               5                   10                  15

Leu Thr Leu Thr Cys Thr Val Ser Gly Phe Ser Leu Ser Ser Tyr Ala

            20                  25                  30

Met Gly Trp Phe Arg Gln Ala Pro Gly Lys Gly Leu Glu Trp Ile Gly

        35                  40                  45

Phe Ile Asn Ile Gly Gly Arg Ala Ser Tyr Ala Ser Trp Ala Lys Gly

    50                  55                  60

Arg Phe Thr Ile Ser Lys Thr Ser Thr Thr Val Asp Leu Lys Met Thr

65                  70                  75                  80

Ser Pro Thr Thr Glu Asp Thr Ala Thr Tyr Phe Cys Ala Arg Gly Arg

                85                  90                  95

Ala Tyr Thr Leu Thr Gly Gly Thr Tyr Thr Arg Ala Phe Asp Pro Trp

            100                 105                 110

Gly Pro Gly Thr Leu Val Thr Val Ser Ser Ala Ser Thr Lys Gly Pro

        115                 120                 125

Ser Val Phe Pro Leu Ala Pro Ser Ser Lys Ser Thr Ser Gly Gly Thr

    130                 135                 140

Ala Ala Leu Gly Cys Leu Val Lys Asp Tyr Phe Pro Glu Pro Val Thr

145                 150                 155                 160

Val Ser Trp Asn Ser Gly Ala Leu Thr Ser Gly Val His Thr Phe Pro

                165                 170                 175

Ala Val Leu Gln Ser Ser Gly Leu Tyr Ser Leu Ser Ser Val Val Thr

            180                 185                 190

Val Pro Ser Ser Ser Leu Gly Thr Gln Thr Tyr Ile Cys Asn Val Asn

        195                 200                 205

His Lys Pro Ser Asn Thr Lys Val Asp Lys Lys Val Glu Pro Lys Ser

    210                 215                 220

Cys Asp Lys Thr His Thr Cys Pro Pro Cys Pro Ala Pro Glu Leu Leu

225                 230                 235                 240

Gly Gly Pro Ser Val Phe Leu Phe Pro Pro Lys Pro Lys Asp Thr Leu

                245                 250                 255

Met Ile Ser Arg Thr Pro Glu Val Thr Cys Val Val Val Asp Val Ser

            260                 265                 270

His Glu Asp Pro Glu Val Lys Phe Asn Trp Tyr Val Asp Gly Val Glu

        275                 280                 285

Val His Asn Ala Lys Thr Lys Pro Arg Glu Glu Gln Tyr Asn Ser Thr

    290                 295                 300

Tyr Arg Val Val Ser Val Leu Thr Val Leu His Gln Asp Trp Leu Asn

305                 310                 315                 320

Gly Lys Glu Tyr Lys Cys Lys Val Ser Asn Lys Ala Leu Pro Ala Pro

                325                 330                 335

Ile Glu Lys Thr Ile Ser Lys Ala Lys Gly Gln Pro Arg Arg Pro Gln

            340                 345                 350

Val Tyr Thr Leu Pro Pro Ser Arg Asp Glu Leu Thr Lys Asn Gln Val

        355                 360                 365

Ser Leu Thr Cys Leu Val Lys Gly Phe Tyr Pro Ser Asp Ile Ala Val

    370                 375                 380

Glu Trp Glu Ser Asn Gly Gln Pro Glu Asn Asn Tyr Lys Thr Thr Pro

385                 390                 395                 400

Pro Val Leu Asp Ser Asp Gly Ser Phe Phe Leu Tyr Ser Lys Leu Thr

                405                 410                 415

Val Asp Lys Ser Arg Trp Gln Gln Gly Asn Val Phe Ser Cys Ser Val

            420                 425                 430

Met His Gly Ala Leu His Asn His Tyr Thr Gln Lys Ser Leu Ser Leu

        435                 440                 445

Ser Pro Gly Lys

    450

<210> 2

<211> 217

<212> PRT

<213> Artificial Sequence

<220>

<223> light chain of anty-C4d mAb2 antibody

<400> 2

Ala Gln Val Leu Thr Gln Thr Ala Ser Pro Val Ser Ala Ala Val Gly

1               5                   10                  15

Ser Thr Val Thr Ile Asn Cys Gln Ser Ser Gln Lys Val Tyr Gly Asn

            20                  25                  30

Asn Asn Leu Ala Trp Tyr Arg Gln Lys Pro Gly Gln Pro Pro Lys Val

        35                  40                  45

Leu Ile Tyr Ala Ala Ser Thr Leu Ala Ser Gly Val Pro Ser Arg Phe

    50                  55                  60

Lys Gly Tyr Gly Ser Gly Thr Gln Phe Thr Leu Thr Ile Ser Asp Leu

65                  70                  75                  80

Glu Cys Asp Asp Ala Ala Thr Tyr Tyr Cys Ala Gly Ala Tyr Gly Gly

                85                  90                  95

Asp Ile Tyr Pro Phe Gly Gly Gly Thr Glu Leu Glu Ile Lys Arg Thr

            100                 105                 110

Val Ala Ala Pro Ser Val Phe Ile Phe Pro Pro Ser Asp Glu Gln Leu

        115                 120                 125

Lys Ser Gly Thr Ala Ser Val Val Cys Leu Leu Asn Asn Phe Tyr Pro

    130                 135                 140

Arg Glu Ala Lys Val Gln Trp Lys Val Asp Asn Ala Leu Gln Ser Gly
145                 150                 155                 160
Asn Ser Gln Glu Ser Val Thr Glu Gln Asp Ser Lys Asp Ser Thr Tyr
                165                 170                 175
Ser Leu Ser Ser Thr Leu Thr Leu Ser Lys Ala Asp Tyr Glu Lys His
            180                 185                 190
Lys Val Tyr Ala Cys Glu Val Thr His Gln Gly Leu Ser Ser Pro Val
        195                 200                 205
Thr Lys Ser Phe Asn Arg Gly Glu Cys
    210                 215

## Description

The invention relates to the application of an anti-C4d monoclonal antibody (mAb2) as a second-line immunotherapeutic for patients exhibiting resistance to the first-line immunotherapy based on anticancer monoclonal antibodies.

Human antibody classes (isotypes) such as IgG1, IgG3, and IgM have the ability to activate the classical complement pathway. This mechanism triggers a cascade on the surface of antibody-coated cells, leading to proteolysis and conformational changes in serum proteins, ultimately forming a membrane attack complex (MAC) that results in osmotic lysis of the target cell. Complement activation by antibodies is considered as one of the effector mechanisms underlying the cytotoxicity of immunotherapeutics, particularly against cancer cells. However, cancer cells have evolved various defense mechanisms against complement attack, including the high expression of membrane-bound complement inhibitors and the recruitment of soluble complement inhibitors from the bloodstream. These proteins facilitate the degradation of early complement cascade proteins, including the active C4b component, into its inactive derivative, C4d.

Patent document EP3191509B1 describes the generation of an antibody recognizing the linear C-terminal neoepitope of the C4d fragment. This specificity ensures that the antibody does not cross-react with the predominant native C4 protein or its C4b fragment in circulation, allowing precise detection of C4d in clinical samples using immunoenzymatic techniques.

Resistance to immunotherapeutics poses a major challenge in oncology and hematology, necessitating the development and using of new generations of anti-cancer drugs. Conventional strategies to enhance therapeutic efficacy have included combining immunotherapy with chemotherapy or engineering new immunotherapeutics with improved properties such as increased target-specific cytotoxicity, optimized pharmacodynamics, or extended half-life. Studies have also suggested the simultaneous or sequential administration of different immunotherapeutics targeting various molecular targets.

A key issue in current state of the art is the selection of appropriate drug combinations for specific indications and the process of efficient modification of existing drugs to enhance their properties. Additionally, introducing new generations of immunotherapeutics targeting the same molecular target as previous therapies requires costly clinical trials before regulatory approval for use.

The objective of this invention was to develop a universal antibody capable of recognizing the antigen which is a marker indicative of prior exposure of cells to therapeutic antibodies and eliminating such cells via the host's innate immune mechanisms. A major advantage of this approach is that the claimed antibody is compatible with a wide range of anti-cancer antibodies, regardless of their molecular targets. The only condition ensuring the broad applicability of such an antibody is the ability of the first-line therapeutic antibody to activate the complement system below the level required to kill the cancer cell. Moreover, antibody preferentially would bind to cells that have survived complement attack, as these cells exhibit C4d as a marker of complement activation.

Unexpectedly, all of the above technical challenges were solved by the monoclonal antibody described in this invention, which is based on a mapped C4d neoepitope sequence. This ensures the specificity of the obtained antibodies for the C4d fragment of the C4 complement protein while minimizing reactivity with the predominant circulating forms of C4b and native C4 protein. Although a partial sequence of this neoepitope was previously described in EP3191509B1, the application of the resulting antibody for second-line immunotherapy has never been proposed.

The subject of this invention is the novel application of an anti-C4d monoclonal antibody (mAb2) containing E430G and/or E345R substitutions as a universal second-line immunotherapeutic agent for the elimination of tumor cells resistant to first-line therapeutic antibodies.

Preferably, the anti-C4d monoclonal antibody described in this invention comprises sequences SEQ ID NO: 1 and SEQ ID NO: 2.

Preferably, the first-line immunotherapy includes treatment for hematologic and lymphatic malignancies.

Preferably, the first-line immunotherapy includes rituximab, daratumumab, and obinutuzumab.

In cases where tumor cells develop resistance to therapeutic antibodies, activation of the classical complement pathway upon antibody binding leaves a footprint - covalently attached C4d fragment on the cell surface. The amount of deposited C4d correlates with the level of complement inhibitors present on the tumor cell surface, which mediate resistance by catalyzing the degradation of C4b to C4d.

A key advantage of this invention is the precise identification of the amino acid sequences forming the variable regions of the anti-C4d monoclonal antibody. This enables the expression of the antibody with predefined properties in widely available *in vitro* protein overproduction systems using synthetic cDNA sequences. Additionally, the detailed characterization of sequences responsible for neoepitope C4d binding allows for modular use of antibody components.

The structural properties of different human antibody classes and their heavy chains are well known in the context of effector mechanisms such as phagocytosis, antibody-dependent cellular cytotoxicity (ADCC), and complement-dependent cytotoxicity (CDC). Similarly, point mutations in the constant region of antibody heavy chains or modifications of antibody glycosylation patterns have been shown to enhance CDC or ADCC activity. This knowledge enables the fusion of the anti-C4d variable region with any chosen antibody isotype or engineered heavy chain to optimize the desired effector function.

### Figure Descriptions

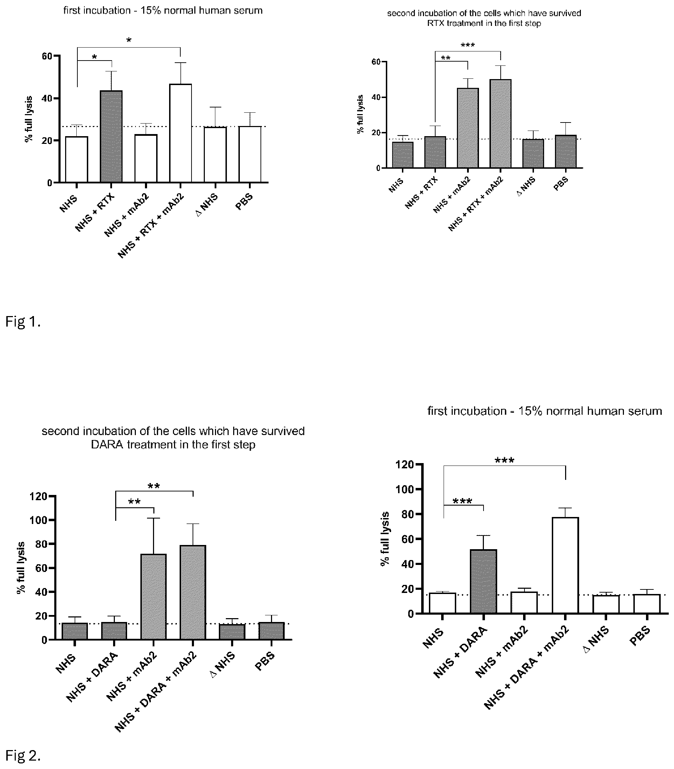

**Fig. 1** shows a two-stage experiment illustrating complement-dependent cytotoxicity (CDC) following a 30-minute incubation of Raji cells with rituximab (RTX), anti-C4d monoclonal antibody (mAb2), or a combination of both in normal human serum (NHS) (left graph). Cells incubated in heat-inactivated serum (ΔNHS) or PBS served as negative controls, indicated by the dashed line. In the second stage (right graph), surviving cells from the first stage (exposed to NHS and RTX) were subjected to a similar experimental procedure. Statistical significance is indicated as *p<0.05, **p<0.01, and ***p<0.001 (one-way ANOVA with Tukey's post hoc test).
**Fig. 2** shows a two-stage experiment illustrating CDC following a 30-minute incubation of Raji cells with daratumumab (DARA), anti-C4d monoclonal antibody (mAb2), or a combination of both in NHS (left graph). Cells incubated in heat-inactivated serum (ΔNHS) or PBS served as negative controls, indicated by the dashed line. In the second stage (right graph), surviving cells from the first stage (exposed to NHS and DARA) were subjected to a similar experimental procedure. Statistical significance is indicated as **p<0.01 and ***p<0.001 (one-way ANOVA with Tukey's post hoc test).

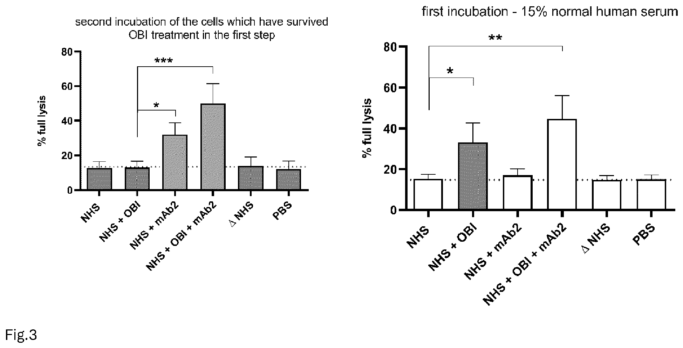

**Fig. 3****:** shows a two-stage experiment illustrating CDC following a 30-minute incubation of Raji cells with obinutuzumab (OBI), anti-C4d monoclonal antibody (mAb2), or a combination of both in NHS (left graph). Cells incubated in heat-inactivated serum (ΔNHS) or PBS served as negative controls, indicated by the dashed line. In the second stage (right graph), surviving cells from the first stage (exposed to NHS and OBI) were subjected to a similar experimental procedure. Statistical significance is indicated as *p<0.05, **p<0.01, and ***p<0.001 (one-way ANOVA with Tukey's post hoc test).

### Example 1

### Monoclonal Anti-C4d Antibody (mAb2)

The monoclonal antibody described in this invention is based on a mapped sequence of the C4d neoepitope. To determine the optimal neoepitope length for the production of monoclonal antibodies targeting C4d, the first step involved screening the specificity of rabbit polyclonal antibodies. As part of an outsourced service provided by Agrisera (Sweden), rabbits were immunized with synthetic peptides corresponding to five- (SSTGR), seven- (TLSSTGR), nine-(NVTLSSTGR), eleven- (GLNVTLSSTGR), and thirteen- (ERGLNVTLSSTGR) amino acid fragments of the C-terminal end of the C4d protein. These peptides were conjugated to keyhole limpet hemocyanin (KLH) via a linker containing two glycine residues (GG).

The rabbits received 200 µg of the immunogen in complete Freund's adjuvant, followed by a booster dose of the same amount in incomplete Freund's adjuvant at week four. Subsequently, they received two injections of 100 µg in incomplete Freund's adjuvant at weeks eight and twelve. The rabbits were exsanguinated at week sixteen, and antibodies were purified from their sera using affinity chromatography. For this purpose, N-terminally biotinylated variants of the immunization peptides (produced by Lipopharm, Poland) were immobilized on HiTrap Streptavidin Sepharose High-Performance columns (Cytiva), and antibody fractions were eluted using 0.1 M glycine buffer at pH 2.5. The eluate was neutralized with Tris-HCl buffer at pH 8.0 and subsequently passed through a second column containing peptides extended by the NGFK amino acid sequence, which represents a segment of the C4b fragment directly adjacent to the C-terminal end of C4d. Unbound fractions from the second column were concentrated to a final volume of 1 ml.

Two independent ELISA assays were performed to evaluate the specificity of the obtained antibodies against the C4d fragment expressed in *E*. *coli* and C4d deposited on the surface of a plate following classical complement pathway activation by immobilized human antibodies diluted in human serum. These assays simultaneously assessed cross-reactivity with C4 and C4b proteins (which were deposited on the plate next to C4d fragment in the first assay and disabled by the application of factor I-depleted serum in the second assay). The most optimal neoepitope sequences, determined to be seven and nine amino acids in length (TVSSTGR and NLTVSSTGR), were then selected for monoclonal antibody production, which was outsourced to Applied StemCell (USA). The monoclonal antibodies were generated using a method based on the cloning of single B lymphocytes derived from Hu Rabbits, a genetically modified rabbit strain that lacks disulfide bridges in the variable regions of its antibodies, rendering them more human-like. Upon receiving 40 monoclonal antibody clones from Applied StemCell, their functionality was assessed using the same ELISA assays applied to the polyclonal antibodies. Three clones, XS41, XS43, and SX4, were selected as the most effective, and Applied StemCell disclosed the nucleotide sequences encoding their heavy and light chains. Competent *E.coli* cells were transformed with plasmids carrying synthetic, sequence-optimized inserts encoding the respective antibody chains for expression in eukaryotic cells. After amplification in bacterial cells, purified DNA was transfected into ExpiCHO cells. Monoclonal antibodies secreted into the culture medium by transfected cells were purified using affinity chromatography on tandemly connected HiTrap columns with immobilized protein A and G (Cytiva). Elution was performed as with the polyclonal antibodies, using 0.1 M glycine buffer at pH 2.5. In ELISA assays, clone XS43 exhibited the lowest cross-reactivity with C4 and C4b while strongly and specifically binding to C4d, even at very low antibody concentrations (0.1 µg/ml). Consequently, the production of chimeric rabbit-human antibodies based on the XS43 sequence was outsourced to Absolute Antibody (UK). Table 1 presents the amino acid sequences of the heavy and light chains of the monoclonal anti-C4d antibody according to this invention, including the precisely defined variable regions responsible for specific antigen binding. Additionally, two point mutations (E345R and/or E430G) were introduced to enhance the antibody's potential for complement-dependent cytotoxicity (CDC). The specificity of the resulting antibody, provisionally named mAb2, for the C4d fragment was confirmed through ELISA assays, and it was subsequently used for *in vitro* studies validating its application as described in this invention (Figures 1, 2, 3).

**Table 1. Amino acid sequences of the heavy and light chains of the anti-C4d mAb2 monoclonal antibody. The variable regions of the heavy and light chains are highlighted in blue, while the constant regions are shown in black. Furthermore, the constant region of the heavy chain, which corresponds to the human IgG1 sequence, contains two substitutions (E345R and/or E430G) highlighted in orange, which were introduced to enhance CDC efficiency.**

| | |
|---|---|
| Heavy chain (SEQ ID NO: 1) | QSVEESGGRLVTPGTPLTLTCTVSGFSLSSYAMGWFRQAPGK GLEWIGFINIGGRASYASWAKGRFTISKTSTTVDLKMTSPTTE DTATYFCARGRAYTLTGGTYTRAFDPWGPGTLVTVSSASTKG PSVFPLAPSSKSTSGGTAALGCLVKDYFPEPVTVSWNSGALTS GVHTFPAVLQSSGLYSLSSVVTVPSSSLGTQTYICNVNHKPSN TKVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIEKT ISKAKGQPRRPQVYTLPPSRDELTKNQVSLTCLVKGFYPSDIAV EWESNGQPENNYKTTPPVLDSDGSFFLYSKLTVDKSRWQQG NVFSCSVMHGALHNHYTQKSLSLSPGK |
| Light chain (SEQ ID NO: 2) | AQVLTQTASPVSAAVGSTVTINCQSSQKVYGNNNLAWYRQK PGQPPKVLIYAASTLASGVPSRFKGYGSGTQFTLTISDLECDDA ATYYCAGAYGGDIYPFGGGTELEIKRTVAAPSVFIFPPSDEQLK SGTASVVCLLNNFYPREAKVQWKVDNALQSGNSQESVTEQD SKDSTYSLSSTLTLSKADYEKHKVYACEVTHQGLSSPVTKSFNR GEC |

### Example 2

### In vitro studies using first-line therapeutic antibody for the treatment of patients with chronic lymphocytic leukemia, e.g., rituximab (RTX)

To investigate the use of the anti-C4d antibody as a remedy for resistance of cancer cells to first-line immunotherapy, a two-step experiment aimed to measure the lysis of cells treated with mAb2 was designed. This experiment used the cells that has survived the contact with the first-line immunotherapeutic agent, such as for the treatment of chronic lymphocytic leukemia (CLL), non-Hodgkin lymphoma, or for the treatment of autoimmune diseases such as pemphigus vulgaris, rheumatoid arthritis, or microscopic vasculitis or granulomatosis with vasculitis (Wegener's granulomatosis).

In the first step, each sample containing 200,000 cells of the human B-lymphocytic lymphoma cell line (Raji) was incubated with calcein-AM solution (1 µl/ml) for 30 min at 37 °C, washed with PBS buffer, and then incubated in 15% normal human serum (NHS) in PBS buffer with added calcium and magnesium for 30 min at 37 °C with shaking (650 rpm). To some samples, rituximab antibody was added at a concentration of 50 µg/ml, to others anti-C4d mAb2 at a concentration of 1 µg/ml, and to some both. The negative control consisted of samples without antibody addition (control without classical complement pathway activator) and cells incubated in heat-inactivated serum, which lacks complement activation capacity. The full lysis control consisted of cells incubated in 30% DMSO solution. The level of lysis (attributable to the level of fluorescence in the supernatant above the cells) was measured using a Synergy H1 microplate reader (BioTek) at an excitation wavelength of 490 nm and an emission wavelength of 520 nm. In the second step of the experiment, cells treated with rituximab and serum that did not undergo lysis in the first step (i.e., resistant to the drug) were collected. They were washed with PBS buffer and then treated with all variants of solutions. After incubation under the same conditions (30 min, 37°C, 650 rpm), the level of cell lysis was measured as described above. The full lysis control was again incubation in 30% DMSO.

*In vitro* experiments demonstrated that the use of anti-C4d antibody against cancer cells that survived the initial contact with rituximab - the therapeutic anti-CD20 antibody used for the treatment of B-cell leukemias and lymphomas - resulted in a significantly greater cytotoxic effect compared to the reuse of the same immunotherapeutic agent. (Fig. 1, right graph, green bars vs NHS + RTX bar). On the other hand, the use of anti-C4d antibodies alone in the first step does not induce cell lysis above the level observed for cells incubated with serum alone (Fig. 1, left graph, NHS+mAb2 bar vs NHS bar). It was shown that the molecular target for anti-C4d antibodies appears only after rituximab interacts with the cancer cell (Fig. 1).

The process of acquiring resistance to immunotherapeutics by cancer cells, as described in the scientific literature, involves a decrease in the expression of the antigen targeted by the anti-cancer antibody and a parallel increase in the expression of complement inhibitors after subsequent drug infusions. Our in vitro experiments mimic repeated drug administration and the selection of resistant cells, which are subsequently eliminated by an antibody targeting the C4d antigen. The antigen appears in amounts proportional to the cells' resistance to the originally applied drug. Importantly, the kinetics of classical complement pathway activation and C4d fragment formation may vary depending on the antibody used in the first step. In the case of rituximab, literature data shows that it is an efficient complement activator, leading to cell lysis of the target cell in a short time. Hence, the small difference in cytotoxicity between the administration of rituximab alone and in combination with the anti-C4d antibody in the first step (Fig. 1, left graph) contrasts with the significant difference observed when both antibodies are administered in the second step of the experiment.

### Example 3

### In vitro studies using the first-line therapeutic antibody for the treatment of multiple myeloma, e.g., daratumumab (DARA)

Further studies were conducted with an analogous two-step experimental procedure as in Example 2, but in this variant of experiment, daratumumab (50 µg/ml) was used as the first-line immunotherapeutic.

When using a therapeutic antibody binding to a different molecular target (CD38), such as daratumumab, the higher cytotoxicity effect with the concurrent administration of anti-C4d antibody is noticeable already in the first step of the experiment (Fig. 2, left graph, NHS + DARA + mAb2 bar). Nevertheless, in this experimental setup, a statistically significant increase in cytotoxicity was also observed after the administration of anti-C4d antibody to cells that survived the first incubation step with human serum and the therapeutic antibody (Fig. 2, right graph, NHS + mAb2 and NHS + DARA + mAb2 bars). This indicates the universal nature of the proposed solution.

### Example 4

### In vitro studies using first-line therapeutic antibody for the treatment of chronic lymphocytic leukemia and follicular lymphoma, e.g., obinutuzumab (OBI)

The experimental procedure for the use of obinutuzumab was identical to that for rituximab and daratumumab (Examples 2-3), with obinutuzumab being used at the same concentration of 50 µg/ml.

In this version of the experiment, where cells were treated with a therapeutic antibody binding to the CD20 molecule other than rituximab, with slower complement activation kinetics , the effect observed was similar to the previous cases (Fig. 3). Despite the fact that obinutuzumab is a weak complement-dependent cytotoxicity (CDC) activator, and regardless of the cytotoxicity observed with the simultaneous administration of the therapeutic antibody and anti-C4d antibody in the first step, the amount of C4d deposited on the surface of the cells in the first step was sufficient to observe cytotoxicity from mAb2 antibody in the second step (Fig. 3, right graph, NHS + mAb2 and NHS + OBI + mAb2 bars). This further emphasizes the versatility of the strategy using anti-C4d antibody, which is not limited to first-line antibodies that efficiently activate the complement system.

## Claims

1. A monoclonal anti-C4d antibody (mAb2) containing E430G and/or E345R substitutions for use in second-line immunotherapy in patients exhibiting resistance to first-line immunotherapy.

2. A monoclonal anti-C4d antibody for use according to claim 1, wherein the antibody contains the sequences SEQ ID NO: 1 and SEQ ID NO: 2.

3. A monoclonal anti-C4d antibody for use according to claim 1, wherein the first-line immunotherapy is immunotherapy for hematologic and lymphatic system tumors.

4. A monoclonal anti-C4d antibody for use according to any of claims 1-3, wherein the first-line immunotherapy includes rituximab, daratumumab, and obinutuzumab.
